# EUROPÄISCHE PATENTSCHRIFT

(11) **EP 1 211 995 B1**
(45) Veröffentlichungstag und Bekanntmachung des Hinweises auf die Patenterteilung: **15.12.2004**
(21) Anmeldenummer: 00956462.6
(22) Anmeldetag: 18.08.2000
(51) Int. Cl.: A61B 18/14

(54) **BIPOLARES MEDIZINISCHES INSTRUMENT**
BIPOLAR MEDICAL INSTRUMENT
INSTRUMENT MEDICAL BIPOLAIRE

(30) Priorität: 27.08.1999 DE 19940689
(43) Veröffentlichungstag der Anmeldung: 12.06.2002
(73) Patentinhaber: Karl Storz GmbH & Co. KG, 78532 Tuttlingen (DE)
(72) Erfinder: BACHER, Uwe, D-78532 Tuttlingen (DE); BLOCHER, Martin, D-78532 Tuttlingen (DE)
(74) Vertreter: Heuckeroth, Volker, Dr.
(86) Internationale Anmeldenummer: PCT/EP2000/008072
(87) Internationale Veröffentlichungsnummer: WO 2001/015614

(56) Entgegenhaltungen:
- EP-A- 0 878 168
- WO-A-99/40861
- DE-A- 4 416 499
- DE-U- 29 604 191
- US-A- 5 293 878
- US-A- 5 295 990
- US-A- 5 743 906

## Beschreibung

Die Erfindung betrifft ein bipolares medizinisches Instrument, mit einem Rohrschaft, mit zumindest zwei Maulteilen, die relativ zueinander beweglich am distalen Ende des Rohrschafts angeordnet und durch ein Gelenk miteinander verbunden sind und jeweils eine Arbeitselektrode unterschiedlicher Polarität ausbilden, wobei jedem Maulteil eine getrennte Stromleitung zugeordnet ist, von denen eine durch ein in dem Rohrschaft angeordnetes, axial bewegliches Kraftübertragungselement gebildet wird, das kraftschlüssig mit zumindest einem der Maulteile verbunden ist.

Ein derartiges Instrument ist aus der DE 196 08 716 C1 bzw. DE U 296 04 191 bekannt.

In der minimal-invasiven Chirurgie wird ein Instrument der eingangs genannten Art zur Durchführung endoskopischer Eingriffe im menschlichen oder tierischen Körper verwendet.

Die beiden Maulteile am distalen Ende des Rohrschafts sind über ein Gelenk miteinander verbunden und können somit durch Betätigen einer Handhabe am proximalen Ende des Rohrschafts geschlossen und geöffnet werden. Je nach dem chirurgischen Verwendungszweck eines solchen Instruments sind die Maulteile als Schneidwerkzeuge mit Schneidkanten ausgebildet, um Gewebe im Körper abzutrennen, oder als Faßwerkzeuge mit entsprechend stumpf aufeinander stoßenden Flächen, um abgetrenntes Gewebe mit den Maulteilen zu fassen und aus dem Körper zu entfernen, oder um ein Organ oder ein Gefäß zu halten, um dieses aus dem Operationsgebiet zu verlegen. Die Maulteile können auch eine Kombination aus einer Schneid- und einer Faßfunktion aufweisen.

Von den beiden Maulteilen ist zumindest eines gelenkig mit dem Rohrschaft verbunden, während das andere Maulteil mit dem Rohrschaft starr oder ebenfalls gelenkig verbunden ist.

Bei der eingangs genannten Art eines medizinischen Instruments ist weiterhin vorgesehen, daß beide Maulteile jeweils eine mit Hochfrequenzstrom beaufschlagbare Arbeitselektrode unterschiedlicher Polarität ausbilden. Beide Maulteile können dementsprechend getrennt voneinander mit jeweils mit einem Pol einer Hochfrequenzspannungsquelle verbunden werden. Durch Beaufschlagen der beiden Maulteile mit bipolarem Hochfrequenzstrom kann einerseits im Falle der Ausbildung als Schneidwerkzeuge die Schneidwirkung durch die thermische Wirkung des Hochfrequenzstroms in dem Gewebe erhöht werden, andererseits kann im Falle einer Ausbildung als Faßwerkzeuge durch die Wärmeentwicklung zwischen den Maulteilen gefaßtes Gewebe koaguliert und eine Blutung des Gewebes damit gestillt werden.

Bei solchen bipolaren Instrumenten besteht stets das Problem einer ausreichenden elektrischen Isolierung zwischen beiden Maulteilen im Bereich des Gelenks, an dem sich beide Maulteile auch im geöffneten Zustand berühren. Beim Beaufschlagen der Maulteile mit Hochfrequenzstrom muß eine elektrische Trennung bestehen, da beide Maulteile an unterschiedliche Potentiale gelegt werden. Das Problem der elektrischen Trennung der beiden Maulteile voneinander stellt sich als um so größer dar, je kleiner ein derartiges Instrument im Bereich der Maulteile und damit im Bereich des Gelenks ausgebildet wird. Eine schmalbauende Ausgestaltung des Instruments im Bereich der Maulteile ist jedoch für die minimal-invasive Chirurgie besonders wichtig.

Bei dem aus der bereits genannten DE 196 08 716 C1 bekannten Instrument sind die beiden Maulteile vollständig aus Metall und somit über ihren gesamten Körper elektrisch leitend ausgebildet. Ein Kraftübertragungselement in Form einer Schub- und Zugstange ist über eine Kniehebelanordnung mit den beiden beweglichen Maulteilen verbunden. Die Schub- und Zugstange dient außerdem als Stromleitung, um eines der beiden Maulteile mit dem einen Pol einer Hochfrequenzspannungsquelle verbinden zu können.

Bei diesem bekannten Instrument wird die elektrische Isolierung der beiden Maulteile dadurch bewerkstelligt, daß in das ansonsten metallisch ausgeführte Gelenk der beiden Maulteile Keramikelemente eingesetzt werden, die also selbst ein Teil des Gelenks bilden. Diese Art der elektrischen Isolierung der beiden Maulteile voneinander im Bereich des Gelenks hat jedoch den Nachteil, daß bei einer Miniaturisierung dieses Instruments auch die Keramikelemente in ihrer Stärke reduziert werden müssen. Da üblicherweise an die Maulteile eine Hochfrequenzspannung in der Größenordnung von 2,5 kV gelegt wird, bedeutet dies, daß bei einer Reduzierung der Stärke der Keramikelemente ein Spannungsdurchschlag durch das Keramikelement hindurch auftreten kann. Ein weiterer Nachteil der Keramikelemente besteht darin, daß an den eingesetzten Keramikelementen beim Bewegen der Maulteile Reibung auftritt, so daß diese im Laufe der Zeit zerrieben werden können. Ein weiterer Nachteil der Ausgestaltung des bekannten Instruments besteht darin, daß aufgrund der vorgesehenen Keramikelemente die Zahl der Teile der Zange im Bereich der Maulteile und somit der konstruktive Aufwand und der Herstellungsaufwand dieses bekannte Instrument nachteilig erhöht ist.

Weiterhin ist aus der DE 43 12 284 A1 ein bipolares medizinisches Instrument bekannt, bei der die Maulteile insgesamt aus Kunststoff bestehen, wobei in den Kunststoff entisolierte Endabschnitte von Stromleitungen eingebettet sind. Die Maulteile sind als Schneidorgane aus Kunststoff ausgebildet, wobei in dem Kunststoff der Maulteile Arbeitselektroden eingebettet sind. An dieser Ausgestaltung ist es nachteilig, daß die Stromzuführung zu den Maulteilen durch separate elektrische Leitungen erfolgt, die im Inneren der Schub- und Zugstange isoliert verlaufen. Die Schub- und Zugstange dient bei dieser Zange somit nicht als Stromleitung. Somit besteht auch bei diesem Instrument wiederum der Nachteil, daß die Zahl der erforderlichen Teile aufgrund der zusätzlichen Stromleitungen erhöht ist. Ein weiterer Nachteil besteht darin, daß die bis in den Kunststoff der Maulteile geführten Endabschnitte der Stromleitungen beim Öffnen und Schließen der Maulteile jeweils auf Biegung beansprucht werden, so daß die Endabschnitte im Laufe der Zeit brechen können und der Stromfluß zu den Elektroden somit unterbrochen ist.

Ein dem vorstehend genannten bekannten Instrument ähnliches Instrument ist aus der WO 99/40861 bekannt. Dieses bekannte medizinische bipolare Instrument weist am distalen Ende des Schafts zwei relativ zueinander bewegliche Maulteile auf, wobei das eine Maulteil beweglich und das andere Maulteil unbeweglich ist. Das bewegliche Maulteil ist mittels einer Feder in eine Stellung vorgespannt, in der es von dem unbeweglichen Maulteil weggeschwenkt ist, d.h. sich in der Offenstellung befindet. Als Betätigungsmechanismus für das bewegliche Maulteil ist ein den Schaft umgebender axial verschiebbarer Rohrschaft vorgesehen, der durch Verschieben in distale Richtung auf die Außenseite des beweglichen Maulteils aufgleitet und dieses dadurch gegen das unbewegliche Maulteil drückt. Das bewegliche Maulteil ist mit dem unbeweglichen Maulteil über ein Stiftgelenk gelenkig verbunden. Die beiden Maulteile bestehen wiederum insgesamt aus Kunststoff, wobei an dem Kunststoff metallische Elektroden befestigt sind. Als Stromzuführung zu den Elektroden dienen wiederum einzelne Drähte, die mit den Elektroden verbunden sind. Somit besteht auch bei diesem Instrument wiederum der Nachteil, daß die Zahl der erforderlichen Teile aufgrund der zusätzlichen Stromleitungen erhöht ist, und daß die als Drähte ausgebildeten Stromzuführungen beim Öffnen und Schließen der Maulteile auf Biegung beansprucht werden.

Der Erfindung liegt daher die Aufgabe zugrunde, ein bipolares medizinisches Instrument der eingangs genannten Art dahingehend weiterzubilden, daß eine sichere Isolierung der Maulteile im Bereich ihres Gelenks ohne erhöhten Teileaufwand und mit geringem konstruktivem Aufwand erreicht wird.

Erfindungsgemäß wird diese Aufgabe bei einem bipolaren medizinischen Instrument der eingangs genannten Art dadurch gelöst, daß zumindest eines der Maulteile zumindest im Bereich des Gelenks einen einstückigen Grundkörper aus einem elektrisch isolierenden Material aufweist, an dem ein die zugehörige Arbeitselektrode bildender elektrisch leitender Maulteileinsatz befestigt ist, der mit der ihm zugeordneten Stromleitung elektrisch leitend verbunden ist.

Erfindungsgemäß ist demnach vorgesehen, zumindest eines der Maulteile mit einem elektrisch isolierenden einstückigen Grundkörper auszugestalten. Ein solcher elektrisch isolierender Grundkörper kann beispielsweise aus einem harten Kunststoff gebildet sein, so daß das Instrument im Bereich seiner Maulteile den hohen Stabilitätsanforderungen genügt. Dadurch, daß nun das Gelenk der Verbindung zwischen dem ersten Maulteil und dem zweiten Maulteil im Bereich des einstückigen isolierenden Grundkörpers vorgesehen ist, wird ohne zusätzliche Bauteile, wie Keramikelemente, eine elektrische Trennung der beiden Maulteile voneinander bewerkstelligt. Der Grundkörper aus isolierendem Material kann besonders massiv ausgebildet werden, so daß die Stabilität dieses Maulteils und die Stabilität des Gelenks so hoch ist, wie wenn die Maulteile vollständig aus Metall gefertigt wären. Diese Ausgestaltung eignet sich sowohl für Instrumente, deren beide Maulteile beweglich sind, als auch für Instrumente, bei denen nur eines der Maulteile beweglich ist. Ein Gelenkstift des Gelenks, der die beiden Maulteile gelenkig miteinander verbindet, kann bei der erfindungsgemäßen Ausgestaltung sogar aus Metall ausgebildet sein, da der aus dem Isolationsmaterial bestehende Grundkörpers eine Stromübertragung auf das andere Maulteil sicher verhindert.

Das zumindest eine Maulteil, das den Grundkörper aus elektrisch isolierendem Material aufweist, kann beispielsweise im Falle von nur einem beweglichen Maulteil das unbewegliche Maulteil sein, während das andere, bewegliche Maulteil, das mit dem Kraftübertragungselement kraftschlüssig verbunden ist, insgesamt metallisch ausgebildet sein kann, ohne daß eine Stromübertragung von diesem beweglichen Maulteil auf das unbewegliche Maulteil durch das Gelenk hindurch auftritt. Auf diese Weise kann ohne weitere Maßnahmen von elektrischen Verbindungen der gewünschte Stromfluß zwischen dem Kraftübertragungselement auf das bewegliche Maulteil erfolgen. Während bei dem aus der DE 196 08 716 C1 bekannten Instrument auch ein isolierendes Keramikelement im Bereich der Anlenkung der Schub- und Zugstange an der Kniehebelanordnung erforderlich ist, entfällt ein solches zusätzliches Isolationsteil bei der vorliegenden Erfindung. Insgesamt wird durch die Erfindung ein besonders einfacher, wenig Teile erfordernder Aufbau des Instruments erreicht.

Somit wird die der Erfindung zugrunde liegende Aufgabe vollkommen gelöst.

In einer bevorzugten Ausgestaltung weist der Grundkörper einen distalen Abschnitt auf, in dem der darin angeordnete Maulteileinsatz außenseitig isoliert eingefaßt ist.

Hierbei ist von Vorteil, daß nur das mit der Arbeitsfläche des Maulteileinsatzes in Berührung kommende Gewebe von dem Hochfrequenzstrom beaufschlagt wird, während unbeteiligtes Gewebe, das im Operationsgebiet mit der Außenseite des Maulteiles in Berührung kommt, durch den Hochfrequenzstrom nicht beeinträchtigt wird. Die Behandlung des Gewebes mit Hochfrequenzstrom kann somit wesentlich gezielter durchgeführt werden.

In einer weiteren bevorzugten Ausgestaltung ist der Maulteileinsatz kraft- und formschlüssig mit dem Grundkörper verbunden.

Die kraft- und formschlüssige Verbindung hat den Vorteil, daß der Maulteileinsatz an dem Grundkörper sicher verankert ist. Die kraft- und formschlüssige Verbindung ist in der Art einer Verzahnung zwischen dem Maulteileinsatz und dem Grundkörper zu verstehen, die ein Abheben des Maulteileinsatzes von dem Grundkörper verhindert. Eine solche Verbindung kann beispielsweise in Form einer T-förmigen Nut in dem Grundkörper und einer dazu komplementären T-förmigen Feder an dem Maulteileinsatz hergestellt werden. Eine kraft- und formschlüssige Verbindung hat den besonderen Vorteil einer hohen mechanischen Beständigkeit, die durch ein Eingießen oder Einbetten des Maulteileinsatzes in dem Grundkörper nicht erreicht werden kann, wie dies im Stand der Technik vorgesehen ist. Außerdem ist eine solche Verbindung besonders temperaturbeständig, da hier keine stoffschlüssige Verbindung wie bei einem Einkleben erforderlich ist.

Herstellungstechnisch besonders bevorzugt ist es, wenn der Maulteileinsatz an dem Grundkörper durch eine Schwalbenschwanz-Verbindung befestigt wird.

Der Maulteileinsatz kann zusätzlich an seinem proximalen Ende mittels einer Schraube oder einem Stift gegen eine Relativverschiebung relativ zu dem Grundkörper gesichert werden.

In einer weiteren bevorzugten Ausgestaltung ist eines der Maulteile unbeweglich mit dem Rohrschaft verbunden, und weist zumindest dieses Maulteil den Grundkörper aus elektrisch isolierendem Material auf.

Wie bereits erwähnt, hat dies den Vorteil, daß das bewegliche Maulteil vollständig aus Metall gefertigt sein kann, wodurch die Stromübertragung von dem Kraftübertragungselement auf das bewegliche Maulteil konstruktiv sehr einfach bewerkstelligt werden kann. Da das unbewegliche Maulteil an dem Rohrschaft befestigt und dementsprechend an seinem proximalen Abschnitt hinsichtlich seiner Abmessung dem Durchmesser des Rohrschafts entspricht, hat die genannte Maßnahme den weiteren Vorteil, daß das unbewegliche Maulteil mit einem Grundkörper aus isolierendem Material mit besonders hoher Steifigkeit ausgebildet werden kann.

Besonders bevorzugt ist es, wenn beide Maulteile jeweils einen Grundkörper aus einem elektrisch isolierendem Material aufweisen, an dem ein die jeweilige Arbeitselektrode der Maulteile bildender elektrisch leitender Maulteileinsatz angeordnet ist.

Diese Maßnahme hat den besonderen Vorteil, daß beide Maulteile zumindest in ihrem proximalen Bereich außenseitig vollkommen isoliert sind, so daß nur das mit den Maulteileinsätzen in Berührung kommende Gewebe mit dem Hochfrequenzstrom beaufschlagt wird.

In einer weiteren bevorzugten Ausgestaltung bildet der Rohrschaft die andere Stromleitung und ist diese isoliert von dem Kraftübertragungselement elektrisch leitend mit dem anderen Maulteil verbunden.

Diese an sich bekannte Maßnahme trägt weiterhin zur konstruktiven Vereinfachung der erfindungsgemäßen Zange bei, da für die Stromzuleitung auf die beiden Maulteile keine zusätzlichen Stromleitungen benötigt werden.

In einer weiteren bevorzugten Ausgestaltung weist zumindest das mit dem Kraftübertragungselement verbundene Maulteil den Grundkörper aus isolierendem Material auf, wobei dann das Kraftübertragungselement an einem proximalen Abschnitt des Grundkörpers angelenkt ist, an dem ein elektrisch leitendes Verbindungselement vorhanden ist, das das Kraftübertragungselement elektrisch leitend mit dem zugehörigen Maulteileinsatz verbindet.

Auch diese Ausgestaltung, nach der das bewegliche Maulteil einen Grundkörper aus einem elektrisch isolierenden Material aufweist, ist vorteilhaft. Der proximale Abschnitt des Grundkörpers aus Isolationswerkstoff wird nämlich durch das elektrisch leitende Verbindungselement zur Stromübertragung auf den Maulteileinsatz überbrückt, ohne daß das elektrisch leitende Verbindungselement auf Biegung beansprucht wird, wenn das Maulteil bewegt wird, da zwischen dem Kraftübertragungselement und dem Verbindungselement eine gelenkige Verbindung und keine flexible Verbindung besteht.

Dabei ist es bevorzugt, wenn durch das Verbindungselement ein Gelenkstift des Gelenks der Verbindung des ersten Maulteils zu dem zweiten Maulteil hindurchgeht.

Hierbei ist von Vorteil, daß das Verbindungselement eine Verstärkung des Gelenkstifts bewirkt und verhindert, daß sich der Gelenkstift in dem nicht-metallischen proximalen Abschnitt der beiden Maulteile freiarbeitet. Der Gelenkstift des Gelenks kann dabei elektrisch isoliert sein, beispielsweise durch eine entsprechende Ummantelung, um im Fall, daß die beiden Enden des Gelenkstifts freiliegen, zu erreichen, daß die gesamte Außenseite des Instruments im Bereich des Gelenks der Maulteile isoliert ausgebildet ist.

In einer weiteren bevorzugten Ausgestaltung ist das Kraftübertragungselement über einen federbelasteten Kontakt in einem proximalen Bereich des Kraftübertragungselements mit einer Stromversorgung verbunden.

Durch die Stromübertragung mittels eines federbelasteten Kontakts ergibt sich der Vorteil einer konstruktiv besonders einfachen Stromübertragung auf das axial bewegliche Kraftübertragungselement, die den weiteren Vorteil besitzt, das die Stromversorgung selbst, beispielsweise in Form eines Steckergehäuses bzw. Steckeranschlusses für ein Stromkabel, am Instrument selbst feststehend angeordnet werden kann.

In bevorzugten Ausgestaltungen dieser Maßnahme ist der federbelastete Kontakt ein Schleifkontakt, beispielsweise ein längliches metallisches Element in Form einer Blattfeder, und/oder weist der Kontakt ein gegen das Kraftübertragungselement mit Federkraft gedrücktes Element, insbesondere eine Kugel, auf. Bei letzterer Ausgestaltung ergibt sich der weitere Vorteil, daß zwischen der Kugel und dem Kraftübertragungselement eine wesentlich geringere Reibung auftritt.

In einer weiteren bevorzugten Ausgestaltung ist der Maulteileinsatz des unbeweglich mit dem Rohrschaft verbundenen Maulteils über ein elektrisch leitendes Drahtelement, das in den proximalen Abschnitt des zweiten Grundkörpers eingebettet ist, elektrisch leitend mit dem Rohrschaft verbunden.

Durch diese Maßnahme wird vorteilhafterweise eine durch die Einbettung in den Grundkörper des unbeweglichen Maulteils von dem Kraftübertragungselement isolierte elektrisch leitende Verbindung zwischen dem Rohrschaft und dem Maulteileinsatz des unbeweglichen Maulteils geschaffen. Da das Maulteil unbeweglich ist, wird das Drahtelement nicht auf Biogung beansprucht. Des weiteren ist das Drahtelement durch die Einbettung in den Grundkörper des unbeweglichen Maulteils gegen mechanische Einflüsse geschützt.

In einer weiteren bevorzugten Ausgestaltung weist ein proximaler Abschnitt des zweiten Maulteils einen Ausbruch mit zwei sich in Längsrichtung erstreckenden Schenkeln auf, zwischen denen der Grundkörper des ersten Maulteils angeordnet und gelenkig mit den Schenkeln verbunden ist.

Diese Maßnahme hat den Vorteil, daß eine besonders schlanke, wenig Platz beanspruchende Bauweise und gehäuseartige Verbindung der beiden Maulteile untereinander erreicht wird.

In einer weiter bevorzugten Ausgestaltung weist ein proximaler Abschnitt des ersten Maulteils einen Gabelabschnitt auf, in den das Kraftübertragungselement eingreift.

Diese Maßnahme hat den weiteren Vorteil, daß die Anlenkung des Kraftübertragungselements an dem beweglichen Maulteil besonders stabil und wenig Platz beanspruchend bewerkstelligt werden kann.

In einer weiteren bevorzugten Ausgestaltung bestehen der Grundkörper des einen Maulteils und/oder gegebenenfalls der Grundkörper des zweiten Maulteils aus einem harten, insbesondere temperaturbeständigen Kunststoff.

Durch diese Maßnahme wird eine besonders hohe Stabilität der Grundkörper der Maulteile erreicht. Wenn der Kunststoff darüber hinaus temperaturbeständig ist, hat dies den Vorteil, daß die Maulteile in einem Autoklaven sterilisiert werden können. Solche harten Kunststoffe sind allgemein bekannt und verfügbar.

Die erfindungsgemäße Zange kann sowohl als Faßinstrument ausgebildet sein, indem einander gegenüberliegende Innenseiten der Arbeitselektroden flächig ausgebildet sind, oder als Schneidinstrument, indem die einander gegenüberliegenden Innenseiten der Arbeitselektroden als Schneidelemente ausgebildet sind. Bei beiden Arten von Instrumenten lassen sich die vorstehend genannten erfindungsgemäßen Ausgestaltungen vorteilhaft nutzen. Bei einer Ausbildung der Arbeitselektroden als Faßwerkzeuge ist es weiterhin bevorzugt, diese im Querschnitt komplementär zueinander V-förmig auszubilden, wodurch ein seitliches Ausweichen des zu fassenden Gewebes vermieden wird.

Im Fall, daß die Arbeitselektroden als Schneidelemente ausgebildet sind, ist es bevorzugt, wenn die Innenseite der einen Arbeitselektrode eine sich längs erstreckende Kerbe und die Innenseite der anderen Arbeitselektrode eine mit der Kerbe schneidend zusammenwirkende Schneide aufweist.

Diese Ausgestaltung der Maulteile ist derjenigen einer Amboßzange ähnlich, mit der hohe Schneidkräfte auf das zu schneidende Gewebe übertragen werden können.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine Gesamtdarstellung einer erfindungsgemäßen bipolaren medizinischen Zange in Seitenansicht;
- Fig. 2: eine perspektivische Ansicht der Zange in Fig. 1 im Bereich der Maulteile in stark vergrößertem Maßstab;
- Fig. 3: einen Längsschnitt durch die Zange im Bereich der Maulteile, wobei die Maulteile ihre Offenstellung einnehmen;
- Fig. 4: eine der Fig. 3 entsprechende Darstellung, in der die Maulteile ihre geschlossene Stellung einnehmen;
- Fig. 5: eine äußerst schematische, teilweise aufgebrochene Darstellung des proximalen Endes der Zange;
- Fig. 6: einen Schnitt entlang der Linie VI-VI in Fig. 3;
- Fig. 7: einen Schnitt entlang der Linie VII-VII in Fig. 3;
- Fig. 8: einen Schnitt entlang der Linie VIII-VIII in Fig. 3;
- Fig. 9: eine Seitenansicht des Maulteileinsatzes in Fig. 8 ohne den zugehörigen Grundkörper;
- Fig. 10: ein weiteres Ausführungsbeispiel für die Formgebung der Maulteileinsätze im Querschnitt;
- Fig. 11: ein noch weiteres Ausführungsbeispiel für einen Maulteileinsatz in Seitenansicht;
- Fig. 12: eine Draufsicht auf den Maulteileinsatz in Fig. 11;
- Fig. 13: ein noch weiteres Ausführungsbeispiel für die Formgebung der Maulteileinsätze in einer perspektivischen Darstellung;
- Fig. 14: ein noch weiteres Ausführungsbeispiel für Maulteileinsätze im Querschnitt;
- Fig. 15: eine der Fig. 5 entsprechende Darstellung des proximalen Endes der Zange gemäß einem weiteren Ausführungsbeispiel; und
- Fig. 16: ein Detail aus Fig. 15 in vergrößertem Maßstab in einer um 90° gedrehten Seitenansicht.

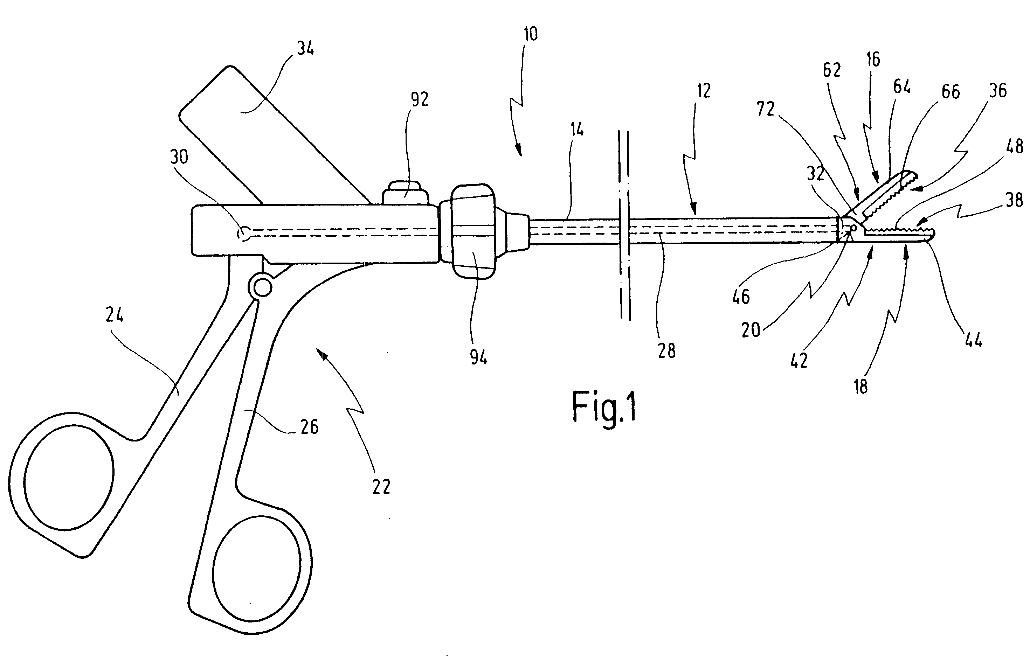

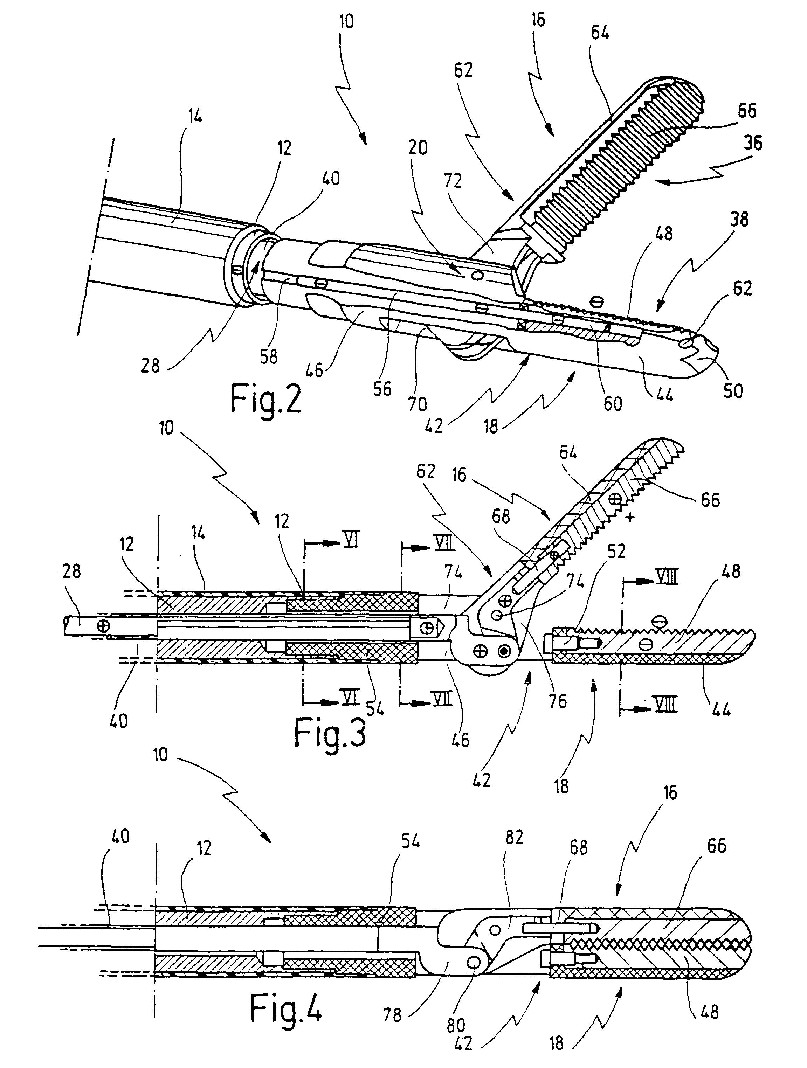

In Fig. 1 ist ein insgesamt mit dem allgemeinen Bezugszeichen 10 versehenes bipolares medizinisches Instrument dargestellt. Einzelheiten des Instruments 10 sind in den Figuren 2 bis 9 dargestellt.

Das Instrument 10 wird im Rahmen der minimal-invasiven Chirurgie zur Behandlung von Gewebe im menschlichen oder tierischen Körper zur Präparation mittels Hochfrequenzstrom verwendet.

Das Instrument 10 ist in dem gezeigten Ausführungsbeispiel ein Faßinstrument bzw. eine Faßzange, wie hiernach noch näher erläutert wird.

Das Instrument 10 weist einen langerstreckten Rohrschaft 12 auf. Der Rohrschaft 12 ist ein als Stromleiter ausgebildetes metallisches Rohr, das von einer isolierenden Umhüllung 14 umgeben ist.

Am distalen Ende des Rohrschafts 12 ist ein erstes Maulteil 16 angeordnet. Am distalen Ende des Rohrschafts 12 ist ferner ein zweites Maulteil 18 angeordnet.

Das erste Maulteil 16 und das zweite Maulteil 18 sind relativ zueinander beweglich. In dem gezeigten Ausführungsbeispiel ist das erste Maulteil 16 beweglich mit dem Rohrschaft 12 und das zweite Maulteil 18 unbeweglich mit dem Rohrschaft 12 verbunden ist.

Das erste Maulteil 16 und das zweite Maulteil 18 sind über ein Gelenk 20 gelenkig miteinander verbunden.

Das Instrument 10 weist weiterhin an ihrem proximalen Ende eine Handhabe 22 auf, die ein erstes, bewegliches Griffteil 24 und ein zweites, unbewegliches Griffteil 26 aufweist.

Um das erste bewegliche Maulteil 16 relativ zu dem zweiten unbeweglichen Maulteil 18 zu bewegen, erstreckt sich zwischen dem beweglichen Griffteil 24 und dem beweglichen Maulteil 16 ein Kraftübertragungselement 28, das hier in Form einer Schub- und Zugstange ausgebildet ist. Das Kraftübertragungselement 28 ist in dem Rohrschaft 12 axial beweglich angeordnet.

Mit seinem proximalen Ende 30 ist das Kraftübertragungselement 28 kraftschlüssig mit dem beweglichen Griffteil 24 verbunden, beispielsweise durch eine Kugelkopf-Kugelpfannen-Verbindung. Mit seinem distalen Ende 32 ist das Kraftübertragungselement 28 mit dem beweglichen Maulteil 16 kraftschlüssig verbunden, wie hiernach noch näher erläutert wird.

Ferner ist am proximalen Ende des Instruments 10 ein schräg stehender Steckeranschluß 34 angeordnet, über den das Instrument 10 an eine nicht dargestellte externe Hochfrequenzspannungsquelle angeschlossen werden kann.

Die beiden Maulteile 16 und 18 bilden jeweils eine Arbeitselektrode 36 und 38 unterschiedlicher Polarität aus, d.h. die Arbeitselektrode 36 ist im Betrieb mit dem einen Pol der Hochfrequenzspannungsquelle verbunden, während die Arbeitselektrode 38 mit dem anderen Pol der Hochfrequenzspannungsquelle verbunden ist.

Das Kraftübertragungselement 28 dient ebenfalls als Stromleitung und ist entsprechend metallisch ausgebildet. Gegenüber dem metallischen Rohrschaft 12 ist das Kraftübertragungselement 28 durch eine isolierende Umhüllung 40, die auf das Kraftübertragungselement 28 aufgebracht ist, isoliert.

Das Kraftübertragungselement 28 dient als elektrisch leitende Verbindung zu dem ersten Maulteil 16, während der Rohrschaft 12 als elektrische Verbindung zu dem zweiten Maulteil 18 dient, wie hiernach noch im einzelnen beschrieben wird.

Mit Bezug auf Figuren 2 bis 4 und 6 bis 9 wird das Instrument 10 im Bereich ihres distalen Endes nun näher beschrieben. Das zweite Maulteil 18 weist einen einstückigen Grundkörper 42 aus einem elektrisch isolierenden Material auf. Dieses Material ist beispielsweise ein harter, temperaturbeständiger Kunststoff, der eine hohe Biegefestigkeit besitzt.

Der Grundkörper 42 weist einen distalen Abschnitt 44 und einen proximalen Abschnitt 46 auf.

An dem distalen Abschnitt 44 des Grundkörpers 42 ist ein Maulteileinsatz 48 angeordnet, der elektrisch leitend ausgebildet ist, und bevorzugt aus Metall besteht. Der Maulteileinsatz 48 bildet die Arbeitselektrode 38.

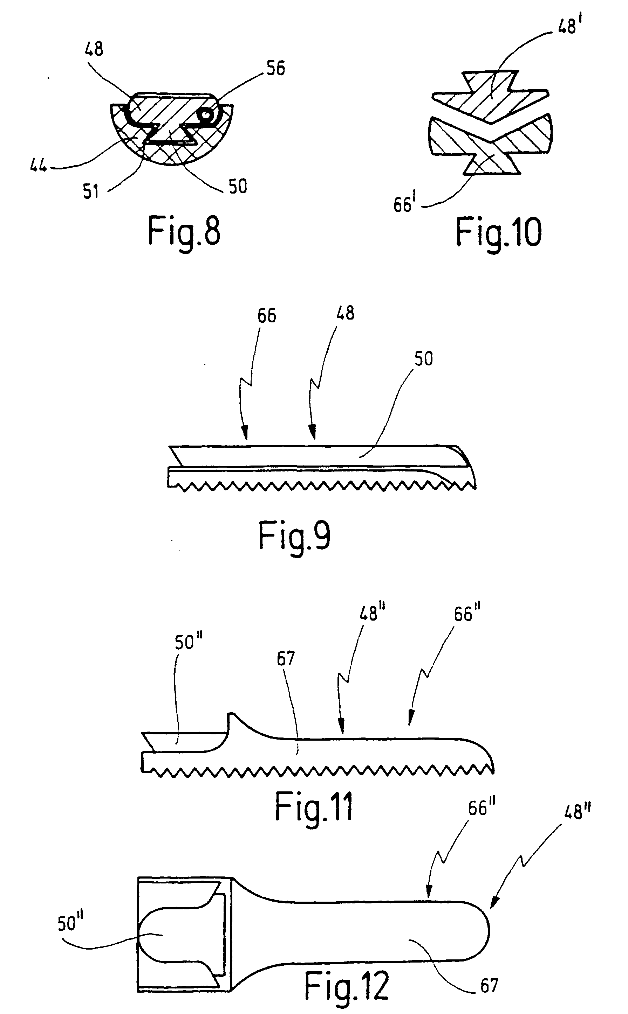

Wie aus Fig. 2 und Fig. 8 hervorgeht, faßt der distale Abschnitt 44 des Grundkörpers 42 den darin angeordneten Maulteileinsatz 48 außenseitig isolierend ein, so daß keine Stromübertragung auf mit der dem Maulteileinsatz 48 gegenüberliegenden Außenseite des Grundkörpers 42 in Berührung kommendes Gewebe erfolgen kann.

Der Maulteileinsatz 48 weist eine schwalbenschwanzförmige Leiste oder Feder 50 auf, mit der der Maulteileinsatz 48 in einer komplementär ausgebildeten Nut 51 des Grundkörpers 42 kraftund formschlüssig in der Art einer Verzahnung mit dem Grundkörper 42 verbunden ist. Die schwalbenschwanzförmige Feder 50 ist ebenfalls elektrisch leitend, d.h. metallisch ausgebildet und ragt bis zur distalen Spitze des Maulteils 18, wo sie nach außen nicht isoliert ist.

An seinem proximalen Ende ist der Maulteileinsatz 48 mittels einer Schraube 52 mit dem Grundkörper 42 verschraubt und somit zusätzlich an dem Grundkörper 42 auch gegen eine Relativverschiebung zu dem Grundkörper 42 gesichert.

Der proximale Abschnitt 46 des Grundkörpers 42 ist über eine einstückig mit diesem verbundene Steckhülse 54, die weiterhin aus demselben elektrisch isolierenden Material besteht, mit dem Rohrschaft 12 im Preßsitz fest verbunden und gegebenenfalls mittels eines Klebstoffes gesichert.

Der Rohrschaft 12 ist mit dem Maulteileinsatz 48 des zweiten Maulteils 18 über ein elektrisch leitendes Drahtelement 56 elektrisch leitend verbunden. Das Drahtelement 56 ist, wie aus Figuren 2, 6 und 7 hervorgeht, in den Grundkörper 42 seitlich der Längsmittelachse des Grundkörpers 42 eingebettet, wozu in dem Grundkörper 42 eine sich darin erstreckende Bohrung vorhanden ist, die an ihrem proximalen Ende 58 in einer offenen Nut endet.

Während Fig. 2 den Grundkörper 42 im von dem Schaft abgezogenen Zustand zeigt, versteht es sich, daß im zusammengesteckten Zustand das Drahtelement 56 mit der Innenseite des Rohrschafts 12 in innigem Kontakt mit diesem verpreßt ist, so daß eine Stromübertragung von dem Rohrschaft 12 auf das Drahtelement 56 gewährleistet ist.

Ein distales Ende 60 des Drahtelements 56 ragt in den Maulteileinsatz 48 hinein (vgl. Fig. 8), so daß von dem Drahtelement 56 eine Stromübertragung auf den Maulteileinsatz 48 gewährleistet ist.

Die zuvor erwähnte Bohrung erstreckt sich dabei entsprechend auch durch den Maulteileinsatz 48 und endet distalseitig in einer Öffnung 62. Die Öffnung 62 dient zur einfacheren Einführung des Drahtelements 56 in den Maulteileinsatz 48 und den Grundkörper 42 und wird anschließend mit einer elektrisch isolierenden Auffüllmasse, beispielsweise einem Klebstoff, versiegelt. Das distale Ende 60 des Drahtelements 56 befindet sich etwa auf halber Länge des Maulteileinsatzes 48.

Das erste Maulteil 16 weist ebenfalls einen einstückigen Grundkörper 62 aus einem elektrisch isolierenden Material auf. Der Grundkörper 62 weist einen distalen Abschnitt 64 auf, in dem wiederum ein elektrisch leitender, die Arbeitselektrode 36 bildender Maulteileinsatz 66 angeordnet ist. Der distale Abschnitt 64 des Grundkörpers 62 und der Maulteileinsatz 66 entsprechen dem distalen Abschnitt 44 des Grundkörpers 42 bzw. dem Maulteileinsatz 48, so daß sich hier eine nähere Beschreibung erübrigt.

Ein Unterschied besteht lediglich darin, daß der Maulteileinsatz 66 an seinem proximalen Ende durch einen Stift 68 an dem Grundkörper 62 zusätzlich fixiert ist.

Der proximale Abschnitt 46 des Grundkörpers 42 des Maulteils 18 weist zwischen der Steckhülse 54 und dem distalen Abschnitt 44 einen Ausbruch 70 auf, in dem ein proximaler Abschnitt 72 des Grundkörpers 62 des ersten Maulteils 16 angeordnet ist. Der Ausbruch 70 ist etwa von rechteckiger Form und bildet zwei Schenkel 74, von denen in Figuren 3 und 4 der linke Schenkel 74 zu sehen ist. Die Schenkel 74 verbinden die Steckhülse 54 und den distalen Abschnitt 44 des Grundkörpers 42 einstückig miteinander und bestehen entsprechend ebenfalls aus demselben elektrisch isolierenden Material.

Das bereits mit Bezug auf Fig. 1 erwähnte Gelenk 20, über das das bewegliche Maulteil 16 mit dem unbeweglichen Maulteil 18 gelenkig verbunden ist, ist nun an dem proximalen Abschnitt 72 des Grundkörpers 62 und dem proximalen Abschnitt 46 des Grundkörpers 42 angeordnet, d.h. proximal hinter den Maulteileinsätzen 48 bzw. 66.

Das Gelenk 20 wird durch einen Gelenkstift 74 gebildet, der durch die Schenkel 74 des Grundkörpers 42 und durch den proximalen Abschnitt 72 des Grundkörpers 62 hindurchgeht. Der Gelenkstift 74 ist zumindest an den Enden, die am Grundkörper 42 offenliegen, elektrisch isoliert.

Der proximale Abschnitt 72 des Grundkörpers 62 des ersten Maulteils 16 ist in Form eines Gabelabschnitts 76 ausgebildet, in den das Kraftübertragungselement 28 eingreift.

Das Kraftübertragungselement 28 weist dazu an seinem distalen Ende einen Anlenkungsabschnitt 78 auf, der in Form eines Winkels ausgebildet ist. Der Anlenkungsabschnitt 78 ist, wie aus Fig. 3 hervorgeht, an das distale Ende des Kraftübertragungselements 28 angesetzt, wobei jedoch auch eine einstückige Ausgestaltung mit dem übrigen Körper des Kraftübertragungselements 28 möglich ist. Der Anlenkungsabschnitt 78 des Kraftübertragungselements 28 ist auf seiner Außenseite nicht isoliert, kann jedoch auch eine isolierende Beschichtung tragen, obwohl dies hier nicht erforderlich ist. Über einen Gelenkstift 80 ist der Anlenkungsabschnitt 78 mit dem proximalen Abschnitt des Grundkörpers 62 kraftschlüssig verbunden, wobei der Gelenkstift 80 elektrisch leitend ist und keine isolierende Umhüllung aufweist.

Um über das Kraftübertragungselement 28 zugeführten Strom auf den Maulteileinsatz 66 zu übertragen, ist zwischen dem Anlenkungsabschnitt 78 und dem Maulteileinsatz 66 ein elektrisch leitendes Verbindungselement 82 angeordnet, das fest mit dem Gabelabschnitt 76 des Grundkörpers 62 verbunden ist, jedoch gelenkig mit dem Kraftübertragungselement 28. Über den elektrisch leitenden Stift 68 ist das Verbindungselement 82 schließlich elektrisch leitend mit dem Maulteileinsatz 66 verbunden.

Der Stromfluß zwischen dem Rohrschaft 12 und dem Maulteileinsatz 48 des zweiten Maulteils 18 erfolgt über das Drahtelement 56. Dieser Stromfluß ist in Fig. 2 mit Minuszeichen angedeutet.

Der Stromfluß zwischen dem Kraftübertragungselement 28 und dem Maulteileinsatz 66 erfolgt über den Anlenkungsabschnitt 78, das Verbindungselement 82, den Stift 68. Dieser Stromfluß ist in Fig. 3 mit Pluszeichen angedeutet. An dem Stromfluß ist außerdem der Gelenkstift 80 beteiligt.

Die Maulteileinsätze 66 und 48 bilden Arbeitselektroden 36 und 38, deren wirksame Arbeitsfläche stumpf aufeinanderstoßende Flächen bilden, so daß die Arbeitelektroden 36 und 38 als Faßwerkzeuge ausgebildet sind, so daß das Instrument 10 als Faßzange Verwendung findet. Um die Griffigkeit der Arbeitselektroden 36 und 38 zu erhöhen, sind die aufeinander stoßenden Arbeitsflächen der Maulteileinsätze 66 und 48 geriffelt ausgebildet, wie insbesondere aus Fig. 2 hervorgeht.

In Fig. 10 ist ein gegenüber den Maulteileinsätzen 48 und 66 abgewandeltes Ausführungsbeispiel dargestellt, bei dem die Maulteileinsätze 48' und 66', die in Fig. 10 ohne die zugehörigen Grundkörper dargestellt sind, Arbeitsflächen aufweisen, die eine im Querschnitt zueinander komplementäre V-Form aufweisen.

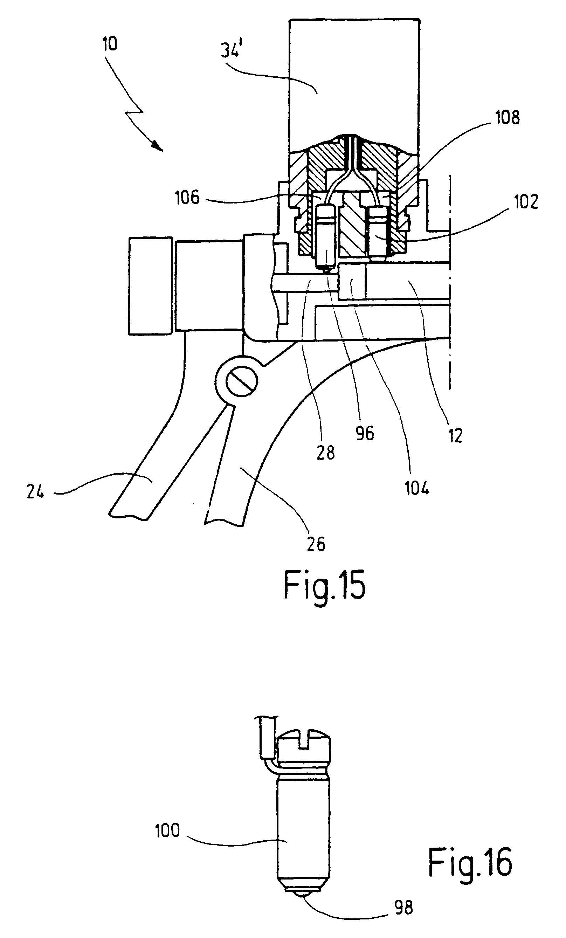

In Fig. 11 und 12 ist ein weiteres Ausführungsbeispiel für eine mögliche Ausgestaltung von Maulteileinsätzen 48'' bzw. 66'', die sich insbesondere bei besonders schlanken Maulteilen eignet.

Bei dieser Ausführung der Maulteileinsätze 48'' und 66" weisen diese nur an ihrem proximalen Ende die schwalbenschwanzförmige Feder 50'' auf, so daß die Maulteileinsätze 48'' und 66'' nur an ihrem proximalen Ende mit einem entsprechenden Grundkörper, wie er in Fig. 11 und 12 nicht dargestellt ist, kraft- und formschlüssig verbunden sind, während ihr distaler Abschnitt 67 nicht von dem Grundkörper eingefaßt wird. Bei dieser Ausführung ist somit der distale Abschnitt 67 der Maulteileinsätze 48" bzw. 66'' außenseitig nicht isoliert.

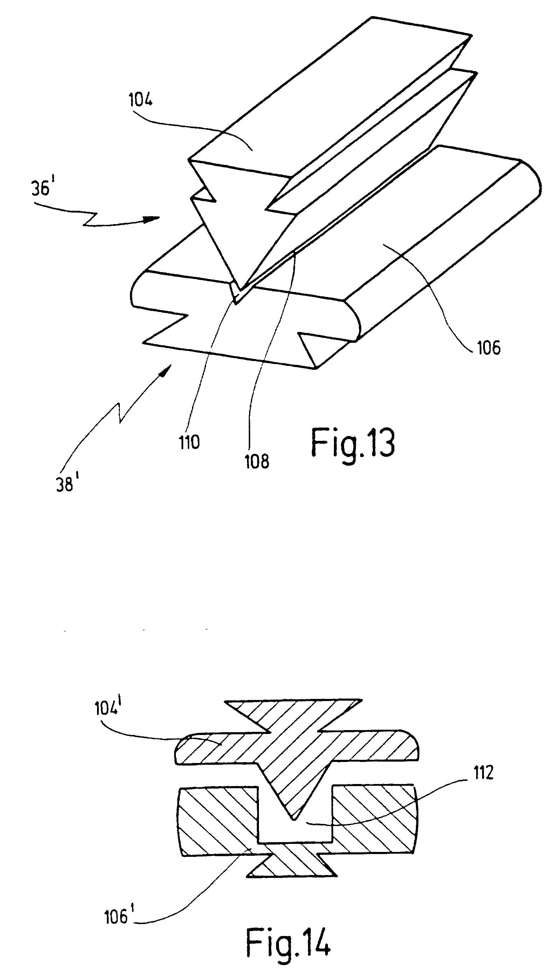

Fig. 13 zeigt ein weiteres Ausführungsbeispiel für Maulteileinsätze 104 und 106 zur Verwendung bei dem Instrument 10. Die Maulteileinsätze 104 und 106 bilden Arbeitselektroden 36' bzw. 38', die als Schneidwerkzeuge ausgebildet sind. Dazu weist der Maulteileinsatz 104 eine Schneide 108 auf, die mit einer Kerbe 110 schneidend zusammenwirkt.

Während die Kerbe 110 in Fig. 13 im Querschnitt etwa V-förmig ausgebildet ist, ist in Fig. 14 ein Ausführungsbeispiel dargestellt, bei dem eine Kerbe 112 eines Maulteileinsatzes 106' im Querschnitt rechteckig ausgebildet ist. Ein mit dem Maulteileinsatz 106' zusammenwirkender Maulteileinsatz 104' ist gegenüber dem Maulteileinsatz 104 dahingehend abgewandelt, daß der Maulteileinsatz 104' sich seitlich etwa genauso breit erstreckt wie der Maulteileinsatz 106'. Beim Schließen der Maulteile, die die Maulteileinsätze 104' und 106' aufweisen, liegen die Maulteileinsätze 104' und 106' an ihren seitlichen Bereichen somit flächig aufeinander.

Mit den in Fig. 13 und 14 gezeigten Maulteileinsätzen 104 und 106 bzw. 104' und 106' wird das Instrument 10 somit als bipolares Elektroschneidinstrument verwendet.

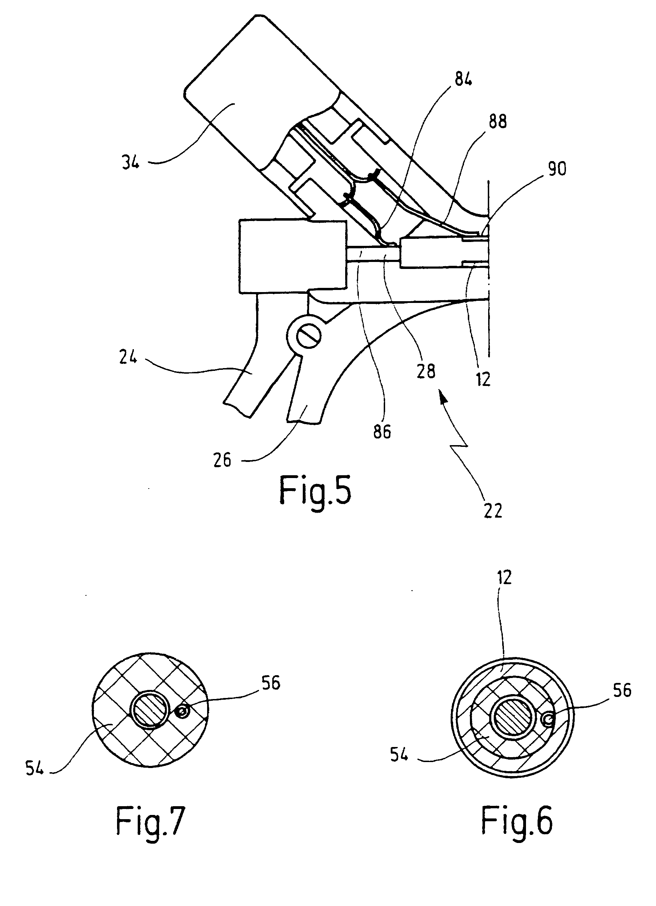

In Fig. 5 ist weiterhin dargestellt, daß die Stromübertragung von dem dem Kraftübertragungselement 28 zugeordneten Steckerpol des Steckeranschlusses 34 über einen als Schleifkontakt 84 ausgebildeten federbelasteten Kontakt erfolgt, der aus einem elastisch gegen einen nicht isolierten Abschnitt 86 des Kraftübertragungselements 28 Federdraht gebildet wird.

Ein zweiter Kontakt 88 drückt gegen ein nicht isoliertes proximales Ende 90 des Rohrschafts 12, um den Rohrschaft 12 mit dem anderen Steckerpol des Steckeranschlusses 34 leitend zu verbinden. Es versteht sich jedoch, daß der Rohrschaft 12 unbeweglich ist, so daß der Kontakt 88 kein Schleifkontakt ist.

In Fig. 15 und 16 ist ein gegenüber Fig. 5 abgewandeltes Ausführungsbeispiel des proximalen Endes des Instruments 10 dargestellt, wobei mit dem Ausführungsbeispiel in Fig. 5 gleiche oder vergleichbare Teile mit den gleichen Bezugszeichen versehen wurden.

Ein Steckeranschluß 34' ist im Unterschied zu dem in Fig. 5 und in Fig. 1 gezeigten Ausführungsbeispiel etwa senkrecht zur Instrumentenachse verlaufend ausgebildet.

Bei diesem Ausführungsbeispiel erfolgt die Stromübertragung auf das Kraftübertragungselement 28 durch einen federbelasteten Kontakt 96, wobei der Kontakt zwischen der entsprechenden Zuleitung und dem Kraftübertragungselement 28 durch eine federbelastete Kugel 98 hergestellt wird, die in einem Gehäuse 100 gehalten und mittels einer in dem Gehäuse 100 angeordneten, nicht näher dargestellten Feder nach unter gegen das Kraftübertragungselement 28 gedrückt wird.

Ein weiterer federbelasteter Kontakt 102, der in seiner Konstruktion dem Kontakt 96 entspricht, dient zur Stromübertragung auf den Rohrschaft 12, wobei am proximalen Ende des Rohrschafts 12 ein Isolierelement 104 zur Stromtrennung zwischen dem Kraftübertragungselement 28 und dem Rohrschaft 12 vorgesehen ist.

Die Kontakte 96 und 102 sind in entsprechenden Aufnahmen 106 und 108 am Steckeranschluß 34' voneinander isoliert aufgenommen.

Wieder mit Bezug auf Fig. 1 ist der Rohrschaft 12 zusammen mit dem Kraftübertragungselement 28 und den Maulteilen 16 und 18 um die Längsachse drehbar, wozu ein Stellrad 94 vorgesehen ist, das mit dem Rohrschaft 12 verbunden ist.

Des weiteren ist eine Raste 92 vorgesehen, über die der Rohrschaft 12 und damit das Kraftübertragungselement 28 von der Handhabe 22 abnehmbar verastet ist.

## Patentansprüche

1. Bipolares medizinisches Instrument, mit einem Rohrschaft (12), mit zumindest zwei Maulteilen (16, 18), die relativ zueinander beweglich am distalen Ende des Rohrschafts (12) angeordnet und durch ein Gelenk (20) miteinander verbunden sind und jeweils eine Arbeitselektrode (36, 38) unterschiedlicher Polarität ausbilden, wobei jedem Maulteil (16, 18) eine getrennte Stromleitung zugeordnet ist, von denen eine durch ein in dem Rohrschaft (12) angeordnetes, axial bewegliches Kraftübertragungselement (28) gebildet wird, das kraftschlüssig mit zumindest einem der Maulteile (16, 18) verbunden ist, **dadurch gekennzeichnet, daß** zumindest eines der Maulteile (16, 18) zumindest im Bereich des Gelenks (20) einen einstückigen Grundkörper (42, 62) aus einem elektrisch isolierenden Material aufweist, an dem ein die zugehörige Arbeitselektrode (36, 38) bildender elektrisch leitender Maulteileinsatz (48, 66; 48'', 66'; 48'', 66''; 104, 106; 104', 106') befestigt ist, der mit der ihm zugeordneten Stromleitung elektrisch leitend verbunden ist.

2. Instrument nach Anspruch 1, **dadurch gekennzeichnet, daß** der Grundkörper (42, 62) einen distalen Abschnitt aufweist, in dem der Maulteileinsatz (48, 66; 48', 66'; 104, 106; 104', 106') außenseitig isoliert eingefaßt ist.

3. Instrument nach Anspruch 1 oder 2, **dadurch gekennzeichnet, daß** der Maulteileinsatz (48, 66; 48', 66'; 48'', 66"; 104, 106; 104', 106') kraft- und formschlüssig mit dem Grundkörper (42, 62) verbunden ist.

4. Instrument nach Anspruch 3, **dadurch gekennzeichnet, daß** der Maulteileinsatz (48, 66; 48', 66'; 48'', 66''; 104, 106; 104', 106') an dem Grundkörper (42, 62) durch eine Schwalbenschwanz - Verbindung befestigt ist.

5. Instrument nach einem der Ansprüche 1 bis 4, **dadurch gekennzeichnet, daß** eines der Maulteile (16, 18) unbeweglich mit dem Rohrschaft (12) verbunden ist, und daß zumindest dieses Maulteil (18) den Grundkörper (42) aus elektrisch isolierendem Material aufweist.

6. Instrument nach einem der Ansprüche 1 bis 5, **dadurch gekennzeichnet, daß** beide Maulteile (16, 18) jeweils einen Grundkörper (42, 62) aus einem elektrisch isolierenden Material aufweisen, an dem ein die jeweilige Arbeitselektrode (36, 38) der Maulteile (16, 18) bildender elektrisch leitender Maulteileinsatz (48, 66; 48', 66'; 48'', 66''; 104, 106; 104', 106') befestigt ist.

7. Instrument nach einem der Ansprüche 1 bis 6, **dadurch gekennzeichnet, daß** der Rohrschaft (12) die andere Stromleitung bildet und isoliert von dem Kraftübertragungselement (28) elektrisch leitend mit dem anderen Maulteil (16, 18) verbunden ist.

8. Instrument nach einem der Ansprüche 1 bis 7, **dadurch gekennzeichnet, daß** zumindest das mit dem Kraftübertragungselement (28) verbundene Maulteil (16) den Grundkörper (62) aus isolierendem Material aufweist, wobei dann das Kraftübertragungselement (28) an einem proximalen Abschnitt (72) des Grundkörpers angelenkt ist, an dem ein elektrisch leitendes Verbindungselement (82) vorhanden ist, das das Kraftübertragungselement (28) elektrisch leitend mit dem zugehörigen Maulteileinsatz (66; 66'; 66''; 104, 104') verbindet.

9. Instrument nach Anspruch 8, **dadurch gekennzeichnet, daß** durch das Verbindungselement (82) ein Gelenkstift (74) des Gelenks (20) der Verbindung des ersten Maulteils (16) mit dem zweiten Maulteil (18) hindurchgeht.

10. Instrument nach einem der Ansprüche 1 bis 9, **dadurch gekennzeichnet, daß** das Kraftübertragungselement (28) über einen federbelasteten Kontakt (84; 96)in einem proximalen Bereich des Kraftübertragungselements (28) mit einer Stromversorgung verbunden ist.

11. Instrument nach Anspruch 10, **dadurch gekennzeichnet, daß** der federbelastete Kontakt ein Schleifkontakt (84) ist.

12. Instrument nach Anspruch 10 oder 11, **dadurch gekennzeichnet, daß** der Kontakt (96) ein gegen das Kraftübertragungselement mit Federkraft gedrücktes Element, insbesondere eine Kugel (98) aufweist.

13. Instrument nach Anspruch 5 und einem der Ansprüche 6 bis 12, **dadurch gekennzeichnet, daß** der Maulteileinsatz (48) des unbeweglich mit dem Rohrschaft (12) verbundenen Maulteils (18) über ein elektrisch leitendes Drahtelement (56), das in den Grundkörper (42) dieses Maulteils (18) eingebettet ist, elektrisch leitend mit dem Rohrschaft (12) verbunden ist.

14. Instrument nach einem der Ansprüche 1 bis 13, **dadurch gekennzeichnet, daß** ein proximaler Abschnitt (46) des zweiten Maulteils (18) einen Ausbruch (70) mit zwei sich in Längsrichtung erstreckenden Schenkeln (74) aufweist, zwischen denen ein proximaler Abschnitt (62) des ersten Maulteils (16) angeordnet und gelenkig mit den Schenkeln (74) verbunden ist.

15. Instrument nach einem der Ansprüche 1 bis 14, **dadurch gekennzeichnet, daß** ein proximaler Abschnitt (62) des ersten Maulteils (16) einen Gabelabschnitt (76) aufweist, in den das Kraftübertragungselement (28) eingreift.

16. Instrument nach einem der Ansprüche 1 bis 15, **dadurch gekennzeichnet, daß** der Grundkörper (42) des einen Maulteils (18) und/oder gegebenenfalls der Grundkörper (66) des anderen Maulteils (18) aus einem harten Kunststoff besteht/bestehen.

17. Instrument nach einem der Ansprüche 1 bis 16, **dadurch gekennzeichnet, daß** einander gegenüberliegende Seiten der Arbeitselektroden (36, 38) flächig ausgebildet sind, so daß die Arbeitselektroden (36, 38) beim Schließen der Maulteile (16, 18) als Faßwerkzeuge zusamenarbeiten.

18. Instrument nach Anspruch 17, **dadurch gekennzeichnet, daß** die Arbeitselektroden (36, 38) im Querschnitt komplementär zueinander V - förmig ausgebildet sind.

19. Instrument nach einem der Ansprüche 1 bis 16, **dadurch gekennzeichnet, daß** einander gegenüberliegende Innenseiten der Arbeitselektroden (36, 38) als Schneidelemente ausgebildet sind, so daß die Arbeitselektroden (36, 38) beim Schließen der Maulteile (16, 18) als Schneidwerkzeuge zusammenarbeiten.

20. Instrument nach Anspruch 19, **dadurch gekennzeichnet, daß** die Innenseite der einen Arbeitselektrode eine sich längserstreckende Kerbe und die Innenseite der anderen Arbeitselektrode eine mit der Kerbe schneidend zusammenwirkende Schneide aufweist.

## Claims

1. A bipolar medical instrument, comprising a tubular shaft (12), at least two jaw parts (16, 18), which are arranged movably, one relative to the other, at the distal end of the tubular shaft (12) and which are connected with one another via a joint (20), and each of which forms a work electrode (36, 38) of different polarity, wherein a separate current line is associated to each jaw part (16, 18), one of which is constituted by an axially movable force transmission element (28) that is arranged in the tubular shaft (12) and is connected with at least one of the jaw parts (16, 18) in force-locking fashion, **characterized in that** at least one of the jaw parts (16, 18) comprises, at least in the region of the joint (20), a single-piece main body (42, 62) made from an electrically insulating material on which an electrically conductive jaw part insert (48, 66; 48", 66'; 48", 66"; 104, 106; 104', 106') is fastened, which forms the respective work electrode (36, 38) and which is connected, in an electrically conductive fashion, to the respective associated current line.

2. The instrument of claim 1, **characterized in that** the main body (42, 62) comprises a distal portion in which the jaw part insert (48, 66; 48', 66'; 104, 106; 104', 106') is enclosed on its outside in insulated fashion.

3. The instrument of claim 1 or 2, **characterized in that** the jaw part insert (48, 66; 48', 66'; 48'', 66''; 104, 106; 104', 106') is connected with the main body (42, 62) in force-locking and in positive fashion.

4. The instrument of claim 3, **characterized in that** the jaw part insert (48, 66; 48', 66'; 48'', 66''; 104, 106; 104', 106') is fastened on the main body (42, 62) by a dovetail connection.

5. The instrument of anyone of claims 1 through 4, **characterized in that** one of the jaw parts (16, 18) is immovably connected with the tubular shaft (12), and at least that jaw part (18) comprises the main body (42) consisting of an electrically insulating material.

6. The instrument of anyone of claims 1 through 5, **characterized in that** each of the two jaw parts (16, 18) comprises a main body (42, 62) made from an electrically insulating material, with an electrically conductive jaw part insert (48, 66; 48', 66'; 48'', 66''; 104, 106; 104', 106'), forming the respective work electrodes (36, 38) of the jaw parts (16, 18), being fastened thereon.

7. The instrument of anyone of claims 1 through 6, **characterized in that** the tubular shaft (12) forms the other current line, and the latter is connected with the other jaw part (16, 18) in electrically conductive fashion, while being insulated from the force transmission element (28).

8. The instrument of anyone of claims 1 through 7, **characterized in that** the main body (62) made from insulating material is provided at least **in that** jaw part (16) which is connected with the force transmission element (28), in which case the force transmission element (28) is hinged on a proximal portion (72) of the main body on which an electrically conductive connection element (62) is provided which then connects the force transmission element (28) with the respective jaw part insert (66; 66'; 66''; 104, 104') in electrically conductive fashion.

9. The instrument of claim 8, **characterized in that** a pivot pin (74) of the joint (20) of the connection of the first jaw part (16) with the second jaw part (18) passes through the connection element (82).

10. The instrument of anyone of claims 1 through 9, **characterized in that** the force transmission element (28) is connected to a current supply via a spring-loaded contact (84; 96) in a proximal region of the force transmission element (28).

11. The instrument of claim 10, **characterized in that** the spring-loaded contact is a wiper contact (84).

12. The instrument of claim 10 or 11, **characterized in that** the contact (96) comprises an element, in particular a ball (98), which is pressed toward the force transmission element in spring-loaded fashion.

13. The instrument of claim 5 and anyone of claims 6 through 12, **characterized in that** the jaw part insert (48) of the jaw part (18), which is immovably connected with the tubular shaft (12), is connected in electrically conductive fashion with the tubular shaft (12) via an electrically conductive wire element (56) which is embedded in the main body (42) of that jaw part (18).

14. The instrument of anyone of claims 1 through 13, **characterized in that** a proximal section (46) of the second jaw part (18) comprises a recess (70) with two legs (74) extending in longitudinal direction, with a proximal portion (62) of the first jaw part (16) being arranged between the legs and being connected with the legs (74) in articulated fashion.

15. The instrument of anyone of claims 1 through 14, **characterized in that** a proximal portion (46) of the first jaw part (16) comprises a forked portion (76) which is engaged by the force transmission element (28).

16. The instrument of anyone of claims 1 through 15, **characterized in that** the main body (42) of the one jaw part (18) and/or the main body (66) consists of the other jaw part (18), if necessary, consists/consist of a hard plastic material.

17. The instrument of anyone of claims 1 through 16, **characterized in that** mutually opposite inner surfaces of the work electrodes (36, 38) are given a planar surface, so that the work electrodes (36, 38) coact as gripping tools when the jaw parts (16,18) are closed.

18. The instrument of claim 17, **characterized in that** the work electrodes (36, 38) are given a mutually complementary V-shaped cross-section.

19. The instrument of anyone of claims 1 through 16, **characterized in that** mutually opposite inner surfaces of the work electrodes (36, 38) are configured as cutting elements, so that the work electrodes (36, 38) coact as cutting tools when the jaw parts (16,18) are closed.

20. The instrument of claim 19, **characterized in that** the inner face of the one work electrode is provided with a notch extending in longitudinal direction, and the inner face of the other work electrode is provided with a cutting edge coacting with that notch in cutting fashion.

## Revendications

1. Instrument médical bipolaire, avec une tige tubulaire (12), avec au moins deux parties de mâchoire (16, 18) qui sont disposées l'une par rapport à l'autre de façon mobile à l'extrémité distale de la tige tubulaire (12) et reliées l'une à l'autre par une articulation (20) et forment chacune une électrode de travail (36, 38) de polarité différente, chaque partie de mâchoire (16, 18) étant assignée à une ligne électrique distincte, dont une est formée par un élément de transmission de force (28) mobile de façon axiale et disposé dans la tige tubulaire (12), qui est relié par friction à au moins une des parties de mâchoire (16, 18), **caractérisé en ce qu'**au moins une des parties de mâchoire (16, 18) comprend au moins dans la région de l'articulation (20) un corps de base en une pièce (42, 62) fabriqué à partir d'un matériau isolant électriquement, auquel est fixée une mise rapportée de partie de mâchoire (36, 38) électroconductrice formant l'électrode de travail correspondante (48, 66 ; 48", 66" ; 48", 66"; 104, 106 ; 104', 106'), qui est reliée de façon électroconductrice avec la ligne électrique qui lui est assignée.

2. Instrument selon la revendication 1, **caractérisé en ce que** le corps de base (42, 62) comprend une section distale, dans laquelle la mise rapportée de la partie de mâchoire (48, 66 ; 48", 66"; 104, 106 ; 104', 106') est enchâssée de façon isolée sur la partie extérieure.

3. Instrument selon la revendication 1 ou 2, **caractérisé en ce que** la mise rapportée de la partie de mâchoire (48, 66 ; 48', 66'; 48", 66"; 104, 106 ; 104', 106') est reliée par friction et par emboîtement au corps de base (42, 62).

4. Instrument selon la revendication 3, **caractérisé en ce que** la mise rapportée de la partie de mâchoire (48, 66 ; 48', 66'; 48", 66"; 104, 106 ; 104', 106') est fixée au corps de base (42, 62) par un assemblage de type queue d'aronde.

5. Instrument selon l'une des revendications 1 à 4, **caractérisé en ce qu'**une des parties de mâchoire (16, 18) est reliée de façon non mobile à la tige tubulaire (12), et **en ce qu'**au moins cette partie de mâchoire (18) comprend le corps de base (42) fabriqué à partir d'un matériau isolant électriquement.

6. Instrument selon l'une des revendications 1 à 5, **caractérisé en ce que** les deux parties de mâchoire (16, 18) comprennent chacune un corps de base (42, 62) fabriqué à partir d'un matériau isolant électriquement, auquel est fixée une mise rapportée (48, 66 ; 48', 66'; 48", 66"; 104, 106 ; 104', 106') électroconductrice formant l'électrode de travail respective (36, 38) des parties de mâchoire (16, 18).

7. Instrument selon l'une des revendications 1 à 6, **caractérisé en ce que** la tige tubulaire (12) forme l'autre ligne électrique et est reliée de façon électroconductrice à l'autre partie de mâchoire (16, 18) et isolée de l'élément de transmission de force (28).

8. Instrument selon l'une des revendications 1 à 7, **caractérisé en ce qu'**au moins la partie de mâchoire (16) reliée à l'élément de transmission de force (28) comprend le corps de base (62) fabriqué à partir d'un matériau isolant, l'élément de transmission de force (28) étant articulé sur une section proximale (72) du corps de base, sur lequel se trouve un élément de raccordement conducteur électrique (82), qui relie l'élément de transmission de force (28) de façon électroconductrice à la mise rapportée de la partie de mâchoire correspondante (66 ; 66' ; 66" ; 104, 104').

9. Instrument selon la revendication 8, **caractérisé en ce que** l'élément de raccordement (82) est traversé par un axe (74) de l'articulation (20) constituant l'assemblage de la première partie de mâchoire (16) à la seconde partie de mâchoire (18).

10. Instrument selon l'une des revendications 1 à 9, **caractérisé en ce que** l'élément de transmission de force (28) est relié à une alimentation électrique via un dispositif de contact sollicité élastiquement (84 ; 96) dans une région proximale de l'élément de transmission de force (28).

11. Instrument selon la revendication 10, **caractérisé en ce que** le dispositif de contact sollicité élastiquement est un dispositif de contact de frottement (84).

12. Instrument selon la revendication 10 ou 11, **caractérisé en ce que** le dispositif de contact (96) comprend un élément pressé contre l'élément de transmission de force avec une force élastique, en particulier une bille (98).

13. Instrument selon la revendication 5 et l'une des revendications 6 à 12, **caractérisé en ce que** la mise rapportée (48) de la partie de mâchoire (18) reliée de façon non mobile à la tige tubulaire (12) est reliée à la tige tubulaire (12) de façon électroconductrice via un fil électroconducteur (56) qui est encastré dans le corps d'outil (42) de cette partie de mâchoire (18).

14. Instrument selon l'une des revendications 1 à 13, **caractérisé en ce que** la région proximale (46) de la seconde partie de mâchoire (18) comprend un évidement (70) avec deux branches (74) s'étendant en direction longitudinale, entre lesquelles une région proximale (62) de la première partie de mâchoire (16) est disposée et reliée de façon articulée aux branches (74).

15. Instrument selon l'une des revendications 1 à 14, **caractérisé en ce que** la région proximale (62) de la première partie de mâchoire (16) comprend une section en fourche (76) dans laquelle s'enclenche l'élément de transmission de force (28).

16. Instrument selon l'une des revendications 1 à 15,
**caractérisé en ce que** le corps de base (42) de l'une des parties de mâchoire (18) et/ou le cas échéant le corps de base (66) de l'autre partie de mâchoire (18) se compose/composent d'une matière plastique dure.

17. Instrument selon l'une des revendications 1 à 16, **caractérisé en ce que** les côtés opposés des électrodes de travail (36, 38) sont réalisés de façon bidimensionnelle de sorte que les électrodes de travail (36, 38), lors de la fermeture des parties de mâchoire (16, 18), coopèrent comme des outils de préhension.

18. Instrument selon la revendication 17, **caractérisé en ce que** les électrodes de travail (36, 38) sont conformées en coupe transversale de façon complémentaire l'une par rapport à l'autre, en forme de V.

19. Instrument selon l'une des revendications 1 à 16, **caractérisé en ce que** les côtés intérieurs opposés des électrodes de travail (36, 38) sont réalisés comme des éléments de coupe, de sorte que les électrodes de travail (36, 38) coopèrent lors de la fermeture des parties de mâchoire (16, 18), comme des outils de coupe.

20. Instrument selon la revendication 19, **caractérisé en ce que** le côté intérieur de l'une des électrodes de travail comprend une encoche s'étendant de façon longitudinale et le côté intérieur de l'autre électrode de travail comprend une arête coopérant de façon coupante avec l'encoche.
